# FASCICULE DE BREVET EUROPEEN

(11) **EP 0 541 874 B1**
(45) Date de publication et mention de la délivrance du brevet: **09.10.1996**
(21) Numéro de dépôt: 91403042.4
(22) Date de dépôt: 14.11.1991
(51) Int. Cl.: A61K 31/70, A61K 47/10, A61K 9/14, A61K 9/16, A61K 9/20

(54) **Compositions à base de diosmine**
Diosmin enthaltende Arzneimittel
Compositions containing diosmin

(43) Date de publication de la demande: 19.05.1993
(73) Titulaire: CORBIERE, Jérôme, F-75116 Paris (FR)
(72) Inventeur: CORBIERE, Jérôme, F-75116 Paris (FR)
(74) Mandataire: Burtin, Jean-François

(56) Documents cités:
- EP-A- 0 275 005
- WO-A-85/03865
- FR-A- 2 577 437
- FR-A- 2 661 610
- FR-M- 6 967

## Description

La présente invention a pour objet de nouvelles compositions pharmaceutiques destinées à la voie digestive à base de flavonoside.

Elle a plus particulièrement pour objet des compositions pharmaceutiques pulvérulentes appropriées pour réaliser une solution ou une suspension fine dans un milieu aveux aisé à ingérer.

Elle a spécifiquement pour objet des préparations pulvérulentes destinées à réaliser une solution ou suspension fine de Diosmine caractérisées en ce que la Diosmine se présente sous une granulométrie particulière et est additionnée d'un agent mouillant et d'un excipient ou un diluant inerte, soluble en milieu aqueux, pharmaceutiquement-acceptable.

On connait depuis plus de 20 ans l'emploi de la Diosmine comme médicament et notamment comme médicament phlebotonique. La diosmine est considérée comme un des agents thérapeutiques des troubles circulatoires veineux les plus efficaces. Toutefois, cette efficacité ne peut être obtenue que par administration répétée et à doses importantes, en raison de la métabolisation rapide de ce principe actif par la flore microbienne présente dans le tube digestif. Las produits d'hydrolyse qui se forment au cours de la métabolisation possèdent une activité nettement amoindrie par rapport au principe actif de départ. C'est pourquoi, il apparait désirable de disposer d'une forme pharmaceutique facile à ingérer et qui permette d'administrer sans problème de déglutition des quantités sensiblement accrues de Diosmine.

En effet, on ne peut augmenter sans limite la taille de comprimés, de dragées ou de gélules, car le volume obtenu ne permet plus une ingestion facile. On cherche de ce fait, à réaliser des formes pharmaceutiques plus concentrées, solubles dans l'eau ou dispersibles dans l'eau. On peut ainsi administrer dans un verre d'eau, une quantité plus importante de Diosmine sous une forme agréable et facile à ingérer.

Ce problème a été résolu selon l'invention en utilisant de la Diosmine d'une granulométrie définie qui facilite sa dispersion et sa résorption, en l'additionnant d'une agent mouillant qui facilite le contact avec l'eau, d'un agent édulcorant non métabolisé, d'un agent de sapidité, d'un agent épaississant et d'un agent aromatisant. On réalise ainsi, une préparation pulvérulente qui se mouille facilement au contact de l'eau et qui forme selon les concentrations une solution ou une dispersion fine, remise facilement en suspension sans qu'il se forme de dépôt au fond du récipient.

En effet, la Diosmine est réputée être très peu soluble dans l'eau. Par le moyen d'une granulométrie fine et par l'adjonction d'un agent mouillant, on réalise un bien meilleur contact entre la Diosmine et l'eau et on obtient beaucoup plus rapidement la dissolution du principe actif et/ou la saturation du milieu aqueux en Diosmine. Il semble que la Diosmine dans ces conditions soit plus soluble dans l'eau éventuellement par formation d'hydrate et surtout que l'état de saturation du milieu aqueux soit obtenu beaucoup plus rapidement.

Selon une caractéristique de l'invention actuellement préférée, l'invention peut encore être définie par les caractéristiques suivantes :
1. la granulométrie de la Diosmine utilisée se situe à 90% au moins entre 100 et 400 microns
2. l'agent mouillant est un agent tensio-actif non ionique du type poly éther comme un polyéthylèneglycol ou un co-polymère oxyde de propylène-oxyde d'éthylène
3. l'agent mouillant est solide, pulvérulent et non cireux
4. l'agent mouillant se trouve présent en quantitiés allant de 0,5 à 5%
5. l'agent édulcorant est un sucre ou un hexitol, non métabolisé ou un édulcorant de synthèse
6. l'agent édulcorant est un sucre non métabolisé comme le finctose
7. l'agent épaississant est une alcoylcellulose comme l'hydroxypropyl méthylcellulose ou la carboxyméthylcellulose ou la croscarmellos sodique
8. l'agent de sapidité est un excipient à saveur acide qui renforce le goût suave des extraits de fruits comme l'arôme orange, l'arôme citron ou l'arôme pamplemousse. On pourra citer à cet égard, l'acide citrique, l'acide tartrique, l'acide gluconique, l'acide sorbique, l'acide malique, l'acide succinique.
   - l'agent aromatisant est un arôme naturel ou synthétique ou semi-synthétique de fruits, éventuellement absorbé sur excipient inerte comme le lactose, le sorbitol ou le sucre en poudre. Parmi les arômes appropriés, on pensera plus particulièrement, en particulier à :
      . l'essence d'orange, de pamplemousse ou de citron
   - la poudre, ainsi parfaitement homogénéisée, peut alors être tamisée et répartie en formes unitaires.

Les formes pharmaceutiques ainsi réalisées contiennent de 0,100 à 0,500 g de Diosmine par prise unitaire.

Les exemples suivants illustrent l'invention sans toutefois la limiter :

### EXEMPLE I

| **Sachets à 0,500 g de Diosmine** | |
|---|---|
| Diosmine micronisée à 100 microns | 500 g |
| Lactose | 356 g |
| Copolymère d'oxyde d'éthylène et d'oxyde de propylène commercialisé sous la marque PLURONIC F68 | 79 g |
| Sorbitol | 30 g |
| Hydroxypropylmethyl cellulose | 15 g |
| Acide tartrique | 4,5 g |
| Arôme naturel orange sur saccharose | 16 g |
| Fructose | 1000 g |
| pour 1000 sachets de poudre pesant chacun 2 g | |

### EXEMPLE II

| **Sachets à 0,300 g de Diosmine** | |
|---|---|
| Diosmine micronisée à 100-200 microns | 300 g |
| Sorbitol pour compression directe, commercialisé sous la dénomination NEOSORB 60 | 660 g |
| Palatinite | 1259 g |
| Aspartam | 4,2 g |
| Copolymère d'oxyde d'éthylène et d'oxyde de propylène | 29 g |
| Acide citrique | 10 g |
| Arôme citron sur saccharose | 10 g |
| pour 1000 sachets de poudre pesant chacun 2 g | |

Palatinite est la marque déposée pour désigner un mélange équimoléculaire d'isomère de α-D-glucopyranosido 1,6-mannitol et de α-D-glucopyranosido 1,6-glucitol, cristallisé avec 2 moles d'eau.

### EXEMPLE III

| **Comprimés à 0,300 g de Diosmine** | |
|---|---|
| Diosmine micronisée à 100 microns | 300 g |
| Caséine formolée commercialisée sous la dénomination Capillact | 45 g |
| Polyvidone excipient | 12 g |
| Palatinite | 420 g |
| Glycine | 25 g |
| Arôme orange q.s | |
| pour 1000 comprimés terminés au poids moyen de 0,800 g | |

Ces comprimés se dispersent au contact de l'eau en fournissant une émulsion fine.

### EXEMPLE IV

### Granulé de Diosmine

On réalise le granulé suivant qui se délite totalement au contact d'un demi-verre d'eau en assurant une suspension laiteuse stable.

Une cuillérée à café de granulé représente 0,500 g de Diosmine.

| | |
|---|---|
| Diosmine | 100 g |
| Polyvidone excipient | 30 g |
| Sucre interverti | 800 g |
| Polyéthylèneglycol 1000 | 45 g |
| Ethylcellulose | 25 g |
| Arôme orange q.s | |

## Revendications

1. Des préparations pharmaceutiques solides destinées à réaliser une solution ou suspension fine de Diosmine caractérisées en ce que la Diosmine, présentée sous une granulométrie particulière, est dispersée dans un agent mouillant et dans un excipient ou diluant inerte soluble en milieu aqueux, pharmaceutiquement-acceptable.

2. Une préparation selon la revendication 1° dans laquelle l'excipient ou le diluant inerte est un de ceux qui conviennent pour l'administration par voie digestive.

3. Une préparation selon la revendication 1° ou la revendication 2° dans laquelle la granulométrie de la Diosmine se situe pour au moins 90%, entre 100 et 400 microns.

4. Une préparation selon l'une des revendications 1 à 3° dans laquelle l'agent mouillant est un agent tensio-actif non ionique du type polyether.

5. Une préparation selon l'une des revendications 1 à 4° dans laquelle l'agent mouillant est un composé solide, pulvérulent et non cireux.

6. Une préparation selon l'une des revendications 1 à 5° dans laquelle l'agent mouillant se trouve présent en quantités allant de 0,5 à 5%.

7. Une préparation selon l'une des revendications 1 à 6° qui contient, en outre, un agent édulcorant non métabolisé.

8. Une préparation selon l'une des revendications 1 à 7° qui contient, en outre, un agent épaississant.

9. Une préparation selon l'une des revendications 1 à 8° qui contient, en outre, un agent de sapidité et un agent aromatisant.

10. Les compositions selon les revendications 1 à 9° présentées sous forme de poudre, de comprimés, de granulés et renfermant de 0,100 à 0,500 g de Diosmine.

## Claims

1. Pharmaceutical solid preparations intended to realize a solution or a fine suspension of Diosmine wherein the Diosmine offered in a particular granulometry is dispersed in a wetting agent and in an inert, soluble in an aqueous medium, pharmaceutically-acceptable carrier or diluent.

2. A preparation according to claim 1, wherein the inert carrier or diluent is one of those suitable for the administration through the digestive way.

3. A preparation according to claim 1 or claim 2, wherein the grain size of Diosmine lies for at least 90% between 100 and 400 microns.

4. A preparation according to one of the claims 1 to 3, wherein the wetting agent is a non-ionic surfactant of the polyether type.

5. A preparation according to one of the claims 1 to 4, wherein the wetting agent is a solid, powdery and not waxy product.

6. A preparation according to one of the claims 1 to 5, wherein the wetting agent is present in amounts ranging from 0.5 to 5%.

7. A preparation according to one of the claims 1 to 6 which further contains a non-metabolized sweetening agent.

8. A preparation according to one of the claims 1 to 7 which further contains a thickening agent.

9. A preparation according to one of the claims 1 to 8 which further contains a flavouring agent and an aromatising agent.

10. The compositions according to claims 1 to 9 offered in the form of powders, tablets, granulates and containing from 0.100 to 0.500 g of Diosmine.

## Patentansprüche

1. Feste pharmazeutische Zubereitungen die für eine Lösung oder eine feine Dispersion von Diosmin bestimmt sind, dadurch gekennzeichnet daß das unter eine besondere Komgröße dargereichte Diosmin in eine Vernetzungsmittel und in einem inerten, ins wässrige Medium lösliche, pharmazeutisch-verträgbare Träger oder Verdünnungsmittel dispergiert wird.

2. Eine Zubereitung nach Anspruch 1, worin der inerte Träger oder Verdünnungsmittel, eine der die für die Abreichung durch den Verdauungstrakt geeignet sind.

3. Eine Zubereitung nach Ansprüche 1 oder 2, worin die Korngröße des Diosmins für mindestens 90% zwischen 100 und 400 mikrons liegt.

4. Eine Zubereitung nach einem der Ansprüche 1 bis 3, worin der Vernetzungsmittel eine non-ionische Tensid des Polyether Typs ist.

5. Eine Zubereitung nach einem der Ansprüche 1 bis 4, worin der Vernetzungsmittel eine feste, pulverige und nicht-wachsartige Verbindung ist.

6. Eine Zubereitung nach einem der Ansprüche 1 bis 5, worin der Vernetzungsmittel anwesend in Anteile von O,5 bis 5 prozent, sich befindet.

7. Eine Zubereitung nach einem der Ansprüche 1 bis 6, die darüberhinaus eine nicht-metabolisierte Süßungsmittel enthält.

8. Eine Zubereitung nach einer der Ansprüche 1 bis 7, die darüberhinaus eine Verdickungsmittel enthält.

9. Ein Zubereitung nach einem der Ansprüche 1 bis 8, die darüberhinaus eine Geschmacksmittel und eine Aromatisierungsmittel enthält.

10. Die Zubereitungen nach Ansprüche 1 bis 9, die in der Form von Pulver, Tabletten, Granulaten dargereicht sind und die von 0,100 bis 0,500 g Diosmin enthälten.
